# EUROPEAN PATENT SPECIFICATION

(11) **EP 4 401 580 B1**
(45) Date of publication and mention of the grant of the patent: **27.05.2026**
(21) Application number: 22789896.2
(22) Date of filing: 19.09.2022
(51) Int. Cl.: A23L 33/12, A23L 33/13, A23L 33/19, A61K 31/14, A61K 31/202, A61K 31/22, A61K 31/7072, A61P 25/00, A61P 25/28

(54) **SOLID COMPOSITION SUITABLE AS A NUTRITIONAL COMPOSITION OR SUPPLEMENT FOR ANIMALS THAT SUFFER FROM BRAIN RELATED DISORDERS, DECLINE OR DISEASES**
FESTE ZUSAMMENSETZUNG, DIE ALS NAHRUNGSZUSAMMENSETZUNG ODER NAHRUNGSERGÄNZUNGSMITTEL FÜR TIERE GEEIGNET IST, DIE AN GEHIRNBEDINGTEN STÖRUNGEN, GEHIRNBEDINGTEM ABBAU ODER GEHIRNBEDINGTEN KRANKHEITEN LEIDEN
COMPOSITION SOLIDE APPROPRIÉE COMME COMPOSITION OU COMPLÉMENT NUTRITIONNEL POUR LES ANIMAUX SOUFFRANT DE TROUBLES, DE DÉCLIN OU DE MALADIES LIÉS AU CERVEAU

(30) Priority: 17.09.2021 EP 21197484
(43) Date of publication of application: 24.07.2024
(60) Divisional application: 26172110.4
(73) Proprietor: N.V. Nutricia, 2712 HM Zoetermeer (NL)
(72) Inventor: PAYNE, Leo Samuel, Auckland, 2022 (NZ); HOLS, Gerrit, 3584 CT Utrecht (NL); COUNOTTE, Danielle Stefanie, 3584 CT Utrecht (NL); GLYN-JONES, Sarah, 3584 CT Utrecht (NL)
(74) Representative: Nederlandsch Octrooibureau
(86) International application number: PCT/EP2022/075941
(87) International publication number: WO 2023/041776

(56) References cited:
- EP-A1- 1 800 675
- WO-A1-2009/002148
- WO-A1-2012/091542
- US-A1- 2007 098 849
- DATABASE GNPD [online] MINTEL; 11 March 2021 (2021-03-11), ANONYMOUS: "Advantage Infant Formula Milk-Based Powder with Iron", XP055896932, retrieved from https://www.gnpd.com/sinatra/recordpage/8554719/ Database accession no. 8554719
- DATABASE GNPD [online] MINTEL; 7 June 2021 (2021-06-07), ANONYMOUS: "Vanilla Flavoured Food for Special Medical Purposes", XP055896826, retrieved from https://www.gnpd.com/sinatra/recordpage/8761077/ Database accession no. 8761077
- SOININEN HILKKA ET AL: "36-month LipiDiDiet multinutrient clinical trial in prodromal Alzheimer's disease", vol. 17, no. 1, 13 September 2020 (2020-09-13), US, pages 29 - 40, XP055896952, ISSN: 1552-5260, Retrieved from the Internet <URL:https://onlinelibrary.wiley.com/doi/full-xml/10.1002/alz.12172> DOI: 10.1002/alz.12172

## Description

### Background of the invention

The present invention relates to a solid composition in powder form suitable as a nutritional composition or supplement for animals, mostly humans, that suffer from brain related disorders, decline or diseases.

The composition that is in powder form contains (i) a uridine source, (ii) a polyunsaturated fatty acid, preferably an omega 3 and (iii) choline, and includes protein source.

Several documents disclose similar compositions that are also disclosed to be suitable for similar treatments.

US20060241077 discloses a composition that contains uridine to improve cognitive function. In one embodiment the nutritional supplement comprises 0.5 g of uridine monophosphate [UMP], 0.3 g of choline, 3.7 g of fish oil, 9.0 g of carbohydrate and 3 g of protein. In the exemplified embodiments the product is a liquid formulation containing choline chloride. It is however suggested that the composition may be formulated as a solid including a granulated solid and separately it is indicated that the choline component may be selected from several salts such as the chloride, bitartrate or stearate salt.

Similarly, US 20150044138 discloses a composition that contains 625 mg UMP, 1500 mg EPA and DHA, and 400 mg choline in a liquid of 125 ml containing in total about 25 g of solids. The document suggests that several choline sources could be chosen such as several choline salts and that the product optionally could also be formulated as a sachet, powder or granule without providing any details on the choices that would have to be made to reduce this into practice. WO2012/091542 exemplifies a similar ready-to-use drink in a trial with elderly suffering from cognitive impairment, focusing on prevention and treatment of frailty, the drink [125 ml] enriched with EPA [300 mg] and DHA [1200 mg], phospholipids [160 mg lecithin], choline [400 mg], UMP [625 mg], antioxidants (40 mg vitamin E, 80 mg vitamin C, 60 µg selenium) B-vitamins (3 µg B12, 1 mg B6, and 400 µg folic acid). The protein is caseinate/WPI. The source of choline is not revealed, but in the other examples with similar products choline chloride is used consistently. In the specification suitable proteins are whey protein, fish protein, protein derived from egg and proteins derived from vegetables. Souvenaid [NV Nutricia; XP055896826], is a flavoured ready-to use 125 ml drink with the above active ingredients and cow's milk proteins in which the choline source according to the pack is choline chloride [Mintel database; "Vanilla flavoured food for special medical purposes" (XP055896826)]. EP1800675 describes a nutraceutical or pharmaceutical composition comprising: a) a lipid fraction comprising at least one of docosahexaneoic acid (DHA), docosapentaenoic acid (DPA) and eicosapentaenoic acid (EPA), b) a protein fraction comprising proteinaceous material from non-human origin which provide at least cysteine and/or taurine, and c) a mineral fraction comprising at least one of manganese and molybdene. WO 2009/002148 discloses the use of a composition comprising (a) one or more [omega]-3 fatty acids selected from DHA, DPA and EPA, (b) uridine or its equivalent, and (c) a methyl donor, in the manufacture of a preparation for the prevention or delay of the onset of dementia in a person having characteristics of a prodromal dementia patient.

US 8,283,336 describes ways to achieve brain health using low-volume administration forms, including tablets, gels, dragees, pills, capsules, granules, pellets, sachets and squeeze packets, with PUFAs such as DHA, nucleotides such as UMP, and tocopherol. The composition is preferably low in proteinaceous content, choline is not mentioned.

There is a desire in the industry to not only provide liquid products but also solid or powder compositions. These will be beneficial as they will reduce transport costs, and are convenient to the consumers in terms of storage, shelf life, and when travelling.

Mastermind (Revive active ) supplement was found to contain DHA, uridine monophosphate, and choline bitartrate. The supplement is promoted to support brain health. However, the product was not found to contain any substantive amount of protein which makes it much less less attractive as a supplement, and less suitable for reconstitution.

In the field of infants, US 2007/098849 discloses infant formulas and corresponding methods of using them to promote retinal health and vision development in infants.

### Summary of the invention:

The invention pertains to a solid composition that is in powder form contains (i) a uridine source, wherein the amount of the sum of all uridine sources, calculated on the basis of the weight of the molar equivalent of uridine monophosphate (UMP), is 0.3-15 g per 100 g dry weight of the composition, (ii) a polyunsaturated fatty acid, preferably an omega 3 fatty acid, (iii) choline bitartrate, (iv) a buffering salt component, and (v) a milk protein source, wherein at least 50% of the choline in the composition is provided by choline bitartrate, wherein the milk protein source is a milk powder and the buffering salt component with a buffering capacity between pH 5 - 8. The buffering salt component preferably contains at least one food-approved salt, more preferably a mixture of food-approved salts, or a food-approved salt and its conjugated acid or base. The buffer preferably maintains the pH in the range of 5 - 8 when the solid composition is dissolved in 1 wt% in water. In the context of the invention, the terms 'solid composition' and 'dry composition' are used interchangeably; the terms 'dry mass', 'dry weight' and 'dry matter' are also used interchangeably. As customary in the field, the dry weight numbers mean that all components but water contribute, it is only the water that is excluded.

The inventors surprisingly found that formulating a solid composition that contains a uridine source, a polyunsaturated fatty acid, a choline source and a protein source, can give rise to several stability issues during and after manufacturing. Choline chloride was found to be very hygroscopic which makes the development of a powder impossible or at least very troublesome. Choline bitartrate has a much reduced hygroscopicity but combining choline bitartrate with a milk protein source results in several other stability issues like protein instability, giving challenges in the preparation process such as phase separation, poor reconstitution, clumping, emulsion instability, and further product quality and stability issues, which especially when using isolated milk proteins such as casein protein or whey protein were quite persistent. It was found that selecting a milk powder instead of processed milk proteins and adding a buffering salt component led to the provision of solid, powdery, composition that overcame the above problems.

The invention provides a solid composition preferably suitable for adult humans to delay or prevent the onset of dementia, such as Alzheimer's Disease. The composition contains (i) a uridine source wherein the amount of the sum of all uridine sources, calculated on the basis of the weight of the molar equivalent of uridine monophosphate (UMP), is 0.3-15 g per 100 g dry weight of the composition, the uridine sources preferably selected from the group consisting of uridine, uridine diphosphate and/or uridine monophosphate, (ii) a polyunsaturated fatty acid, preferably an omega-3 fatty acid, (iii) choline bitartrate, (iv) a buffering salt component, and (v), a milk protein source, wherein at least 50% of the choline in the composition is provided by choline bitartrate, wherein the milk protein source is a milk powder, and the buffering salt component containing at least one food approved salt, with a buffering capacity between pH 5 - 8, the buffering salt component preferably containing a mixture of food-approved salts, or a food-approved salt and its conjugated acid or base.

The composition is not an infant milk formula powder or follow-on formula milk powder.

The invention also provides a process for preparing the solid composition by either dry blending the components (i) to (v) in dry form, or by bringing the components (i) to (v) together in an aqueous solution and subsequently drying the obtained solution by a drying step, that is preferably an evaporation, spray or freeze drying step.

The invention also provides the solid composition in powder form for use in adult humans to delay or prevent the onset of dementia, such as Alzheimer's Disease The composition is preferably administered to the adult in reconstituted form.

The following preferred embodiments are listed here:
- The solid composition as described herein, wherein the polyunsaturated fatty acid is EPA, DHA and/or DPA, preferably EPA and/or DHA, most preferably at least DHA.
- The solid composition as described herein, wherein the uridine source is one or more of uridine, or an uridine salt, wherein the salt is preferably a uridine phosphate salt, preferably uridine, uridine diphosphate and/or uridine monophosphate.
- The solid composition as described herein, wherein the buffering salt component comprises one or more salts of the group of citrate salts, acetate salt and hydrogen phosphate salts.
- The solid composition as described herein, wherein the milk protein source is skim milk powder.
- The solid composition as described herein, wherein the composition comprises 1- 10 g protein per serving, more preferably 2 - 6 g per serving.
- The solid composition as described herein, wherein the composition comprises more than 0.1 g choline per serving, preferably 0.1 to 2 g choline per serving, more preferably 0.2 to 2 g choline per serving, most preferably 0.2 to 1 g choline per serving.
- The solid composition as described herein, wherein at least 70%, more preferably at least 85 %, most preferably at least 95 % of the choline in the composition is provided by choline bitartrate.
- The solid composition as described herein, wherein DHA is administered in in an amount of at least 0.5 g per 100 g, more preferably 0.5 - 5 g per serving, most preferably 0.75 - 2 g per serving.
- The solid composition as described herein, wherein the composition is provided in the form of a canned powder or powder sachet, suitable for oral administration, wherein the active ingredients are present in therapeutically effective amounts.
- The solid composition as described herein, wherein the buffering salt composition maintains the pH in the range of 5-8 when the solid composition is dissolved in 1 wt% in water.
- The solid composition as described herein, wherein the composition is provided in powder form comprised in a can or box, or in a sachet.
- The solid composition of any one of the preceding claims, wherein the composition contains the uridine source in an amount of 1 to 5 wt% on dry matter, and/or DHA and/or EPA in an amount of 2.5 to 25 wt% on dry matter, and/or the choline bitartrate in an amount of 1 to 5 wt% on dry matter.
- The solid composition as described herein, further comprising vitamins B6, B9 and B12, preferably further comprising vitamins B6, B9 and B12, and vitamins C and E, more preferably further comprising vitamins B6, B9 and B12, and vitamins C and E, and selenium.

Being able to provide a dry powder composition containing the mentioned components results in a generally available alternative product that is suitable to replace liquid formulation. The solid composition is very easy to mix with for example water or other (aqueous) liquids; it is preferably a substantially nutritionally complete liquid, wherein with the term 'substantially nutritionally complete' it is preferably understood that a serving of the composition preferably provides at least 80% of the recommended daily values of carbohydrates, protein, fat, vitamins and minerals. The powder composition has good anticaking properties and is free flowing. Reconstitution properties are unexpectedly good as well as sensory properties.

As an additional advantage, distribution of the solid product is much easier, does not require the transport of volumes of liquid, especially in more remote parts of the world.

### Detailed description of the invention

Here below, the numbers per serving are preferably based on a dry mass of 20 - 40 g, more preferably 25 - 35 g. A serving is the predetermined amount of dry solid that is administered to the person in need thereof and/or corresponds to the amount that is prescribed on the package or in accompanying instructions. Such a serving is preferably reconstituted with water or a drink using a dilution factor of 4x. i.e. 25 g per 100 ml.

Most preferably, in the context of the invention, when mention is made of a 'serving', it is understood to mean a solid dosage unit. Dosage units are defined as physically identifiable separate units, and will be detailed below. A serving preferably provides a daily dosage meaning it is recommended to provide one serving per day.

### (i) a uridine source

The uridine source according to the invention can be uridine or cytidine, as free base or as a salt, as an ester, as a phosphate derivative, like UMP, UDP, UTP, CMP, CDP or CTP, as cytosine, and as choline derivative, e.g. as citicoline. Although cytidine is a precursor of uridine, it is more efficient and effective to include uridine or an uridine salt in the composition or for use according to the invention, because it passes the blood brain barrier more easily. In a preferred embodiment the uridine source is one or more of uridine or an uridine salt, wherein the salt is preferably a uridine phosphate (such as uridine monophosphate or uridine diphosphate) salt. The uridine source is preferably uridine, uridine diphosphate and/or uridine monophosphate. Uridine monophosphate or uridine are particularly preferred. Preferably at least 50% of the uridine is provided by UMP.

The composition comprises, per 100 g dry solid, 0.3-15 g, preferably 0.6-7.5 g, more preferably 0.75-3 g of the sum of all of the aforementioned uridine sources (calculated taking the molar equivalent to the UMP amount (molecular weight [MW] 324 Dalton)). That is, the contribution of x gram of cytidine monophosphate is first calculated in terms of UMP molar equivalent (x divided by the MW of CMP) and then recalculated in the corresponding weight of UMP (multiplying *x*/MW_{CMP} with MW_{UMP}). The composition preferably comprises, per 100 g dry solid, 0.3-15 g, preferably 0.6-7.5 g, more preferably 0.75-3 g of the sum of uridine, uridine diphosphate and/or uridine monophosphate.

The composition preferably comprises an amount of the sum of all uridine sources (calculated taking the molar equivalent to the UMP amount (molecular weight 324 Dalton)) of 0.1-5 g, preferably 0.2-2.5 g, more preferably 0.25-1 g per serving. In terms of daily dosage, the composition provides in the range of 0.1-5 g, preferably 0.2-2.5 g, more preferably 0.25-1 g per day. Preferably the above numbers per serving, per 100 g dry solid and per daily dosage apply to the sum of UMP and uridine.

### (ii) a polyunsaturated fatty acid, preferably an omega 3 or omega 6 fatty acid,

The polyunsaturated fatty acid may be a ω-6 polyunsaturated fatty acid (ω-6 PUFA) or a ω-3 polyunsaturated fatty acid (ω-3 PUFA). Preferably it is a ω-3 PUFA, more preferably selected from the group of docosahexaenoic acid (22:6 ω-3; DHA), docosapentaenoic acid (22:5 ω-3; DPA) and eicosapentaenoic acid (20:5 ω-3; EPA). Useful sources include fish oil, algae oil, eggs lipids and genetically modified organisms.

Preferably, the composition of or for use according to the invention comprises at least DHA, preferably DHA and EPA. More preferably, the combination comprises DHA and at least one precursor of DHA selected from EPA and DPA. More preferably, the composition of or for use according to the invention comprises DHA and EPA. In a further aspect, the composition of or for use according to the invention preferably contains EPA. EPA is converted to DPA (ω-3), increasing subsequent conversion of DPA (ω-3) to DHA in the brain. Hence, the composition of or for use according to the invention preferably also contains a significant amount of EPA, so to further stimulate in-vivo DHA formation.

The ω-3 PUFAs, in particular the LCPUFAs, more in particular DHA, DPA and EPA, may be provided in any form such as, triglycerides, diglycerides, monoglycerides, free fatty acids or their salts or esters, glycerol ethers, lipoproteins, ceramides, glycolipids or combinations thereof. Preferably, the composition used or for use according to the invention comprises at least DHA in triglyceride form. In yet another preferred embodiment of the present invention, the PUFA is present as an encapsulated PUFA. These are commonly available to the skilled person. An advantage of employing an encapsulated PUFA is that these are in cases easier to dry mix with other components. Examples of encapsulated PUFAs are for example available from for instance Friesland Brands, Numega, DSM and BASF.

The total daily dosage of DHA+EPA taken together is in the range of 0.25 - 5 g, preferably 0.5 - 5 g, more preferably 0.75 - 2.5 g, per serving, or in the range of 0.75-15 g, preferably 1.5 - 15 g, more preferably 2.25 - 7.75 g per 100 g dry solid. In terms of daily dosage, the composition preferably provides in the range of 0.25 - 5 g, preferably 0.5 - 5 g, more preferably 0.75 - 2.5 g, per day. DHA is preferably administered in in an amount of at least 0.5 g per serving, more preferably 0.5 - 5 g per serving, most preferably 0.75 - 2 g per serving. Alternatively, DHA is preferably administered in in an amount of at least 1.5 g per 100 g dry solid, more preferably 1.5 - 15 g, most preferably 2.25 - 6 g. In terms of daily dosage, the composition preferably provides in the range of at least 0.5 g, more preferably 0.5 - 5 g, most preferably 0.75 - 2 g per day. Where the PUFAs are provided in encapsulated form, the above numbers apply to the DHA and EPA components therein.

The DHA and EPA are preferably provided in the form of marine or algae oil, preferably as a fish oil.

### (iii) choline bitartrate

The choline source in the composition of the invention is a choline bitartrate salt. In embodiments it is possible to add an additional choline salt such as choline chloride, choline butyrate or choline stearate, preferably choline chloride or choline stearate, albeit that at least 50%, more preferably at least 70%, even more preferably at least 85 %, most preferably at least 95 % of the choline in the composition is provided by choline bitartrate. It is preferred that less than 15 %, more preferably less than 5 % of the choline in the composition is provided by choline chloride. In a preferred embodiment, at least 85 %, most preferably at least 95 % of the choline in the composition is provided by choline bitartrate, and less than 5 % of the choline in the composition is provided by choline chloride.

The composition preferably comprises more than 0.1 g choline per serving, preferably 0.1 to 2 g choline per serving, more preferably 0.2 to 2 g choline per serving, even more preferably 0.2 to 1 g choline per serving. In terms of dry weight, the composition preferably comprises more than 0.3 g choline per 100 g dry solid, more preferably 0.3 to 6 g choline, even more preferably 0.6 to 6 g choline, most preferably 0.6 to 3 g choline. In terms of daily dosage, the composition preferably provides more than 0.1 g choline, preferably 0.1 to 2 g choline, more preferably 0.2 to 2 g choline per day, even more preferably 0.2 to 1 g choline per day. It is preferred that protein and choline are present in the composition in a weight ratio between 5:1 and 20:1, preferably 5:1 - 10:1. The above numbers are based on choline, the amounts of choline equivalents or sources can be calculated taking the molar equivalent to choline into account, based on the molar mass of 104 g/mol choline.

### (iv) a buffering salt component

The buffering salt component has a buffering capacity in the range of pH 5-8, preferably 5-7. It preferably contains at least one food-approved salt, more preferably a mixture of food-approved salts, or a food-approved salt and its conjugated acid or base. In preferred embodiments the buffering salt component maintains the pH at a value of 5 - 8 when the composition of the invention is dissolved as a 1 wt% solution in water. Preferably, the buffering salt component is a citrate buffering salt, acetate buffering salt or a phosphate buffering salt, or mixtures thereof, and/or mixtures with their conjugated bases or acids. The buffering salt component preferably comprises one or more salts that are citrate salts, monohydrogen phosphate salts, dihydrogen phosphate salts or acetate salts, and their corresponding conjugated acids or bases. In a preferred embodiment a mixture of an acetate salt or acetic acid, and/or a citrate salt or citric acid are employed. Phosphate buffering agents may not be preferred when the composition already comprises significant amounts of uridine phosphates, thus controlling the amount of phosphate administered. Even more preferred an acetate salt and a citrate salt, preferably with their corresponding conjugated acids, are employed. The cation may be selected to preference, monitoring the mineral levels. It is preferred to use sodium, potassium, magnesium and calcium salts. Most preferred choices are acetate - citrate buffers, such as a buffering combination of tripotassium citrate and magnesium acetate.

In yet another preferred embodiment the amount of buffering salt component is 0.2 to 10 wt% on dry weight, preferably 0.5-7 wt%.

The pH of the composition when determined for a 1wt% solution in water is preferably 5 to 8, even more preferably it is 5 to 7. It is within the general skills of an expert to find a good choice for a buffering salt component.

### (v) a milk protein source

The composition of the invention contain a milk protein source that is a milk powder. The milk powder is defined as a dried milk powder, such as a whole milk powder, low fat milk powder or skim milk powder, and is preferably skim milk powder. Skim milk is a concept known to the skilled person in the art, and is preferably understood milk but with a reduced fat content, typically less than 0.1 wt% fat (compared to 3-4 % fat in whole milk). A suitable commercial source of skim milk powder preferably comprises 30 - 40 wt% protein, typically about 35 wt%. The proteins in the milk powder are preferably in intact form. The milk is preferably a bovine milk, comprising a mixture of casein and whey protein, characterized by a typical 80:20 weight ratio. The protein fraction of the composition preferably comprises less than 20 wt%, preferably less than 10 wt%, most preferably less than 5 wt% of proteins other than the proteins provided by the milk powder, including small amounts of additional casein, caseinate or whey protein. The composition preferably comprises 1- 10 g protein per serving, more preferably 2 - 6 g. Alternatively, the composition preferably comprises 3-30 g per 100 g dry solid, more preferably 5 - 18 g per 100g dry solid. In terms of daily dosage, the composition preferably provides 1-10 g protein, more preferably 2 - 6 g per day.

In a preferred embodiment the composition contains the uridine source in an amount of 1 to 5 wt% on dry matter.

In a further preferred embodiment the composition contains the choline bitartrate in an amount of 1 to 5 wt% on dry matter.

The preferred amount of milk protein is between 15 and 25 wt% on dry weight.

### Further components

The composition according to the invention in addition may contain one or more of vitamin B6, vitamin B9, vitamin B12, selenium, vitamin C, vitamin E (tocopherol), phospholipids, flavoring agents, carbohydrates (such as sugars, maltodextrin or lactose).

The composition preferably further comprises vitamins B6, B9 and B12. The term 'vitamin B6' includes all food-grade vitamin B equivalents, such as there are pyridoxine, pyridoxal, pyridoxamine, and pyridoxine hydrochloride). The term 'vitamin B9' includes folic acid and folate salts, including folic acid, folinic acid, methylated, methenylated and formylated forms of folates. The term 'vitamin B12' includes all food-grade cobalamin equivalents. The composition more preferably further comprises vitamins B6, B9 and B12, and vitamins C and E. The composition even more preferably further comprises vitamins B6, B9 and B12, and vitamins C and E, and selenium. The composition most preferably further comprises vitamins B6, B9 and B12, and vitamins C and E, and selenium, phospholipids, and even more preferably also vitamins A and D.

Vitamin B6 is preferably administered to provide an amount of 0.2 - 5 mg, preferably 0.5 - 3 mg, based per serving or per day. Alternatively, Vitamin B6 is preferably administered in an amount of 0.6 - 15 mg, preferably 1.5 - 9 mg per 100 g dry solid. Vitamin B9 is preferably administered to provide an amount to provide 50 to 1000 µg, in particular in the range of 100 to 1000 µg, more in particular in the range of 200 to 800 µg per serving or per day. Alternatively, vitamin B9 is preferably administered in an amount of 150 to 3000 µg, in particular in the range of 300 to 3000 µg, more in particular in the range of 600 to 2400 µg per 100 g dry solid. The amount of vitamin B9 equivalents is calculated in terms of the molar equivalent of folate. Vitamin B12 is preferably administered to provide an amount in the range of 0.5 to 20 µg, in particular in the range of 1 to 10 µg per serving or per day. Alternatively, vitamin B12 is preferably administered in an amount in the range of 1.5 to 60 µg, in particular in the range of 3 to 30 µg per 100 g dry solid.

Vitamin C, or a functional equivalent thereof, may be present in an amount in the range of 50 to 2000 mg, in particular in the range of 100 to 1000 mg, more in particular in the range of 200 to 700 mg per day or per serving. Alternatively, Vitamin C, or a functional equivalent thereof, may be present in an amount in the range of 150 to 6000 mg, in particular in the range of 300 to 3000 mg, more in particular in the range of 300 to 2100 mg per 100 g dry solid.

Tocopherol and/or an equivalent thereof (i.e. a compound having vitamin E activity) may be provided in an amount in the range of 10 to 300 mg, in particular in the range of 30 to 200 mg, more in particular in the range of 30 to 100 mg per day, to prevent oxidative damage. In one embodiment, tocopherol and/or equivalent is present in an amount in the range of 10 to 300 mg, in particular in the range of 30 to 200 mg, more in particular in the range of 30 to 100 mg per serving. Alternatively, tocopherol and/or equivalent is present in an amount in the range of 30 to 900 mg, in particular in the range of 90 to 600 mg, more in particular in the range of 90 to 300 mg per 100 g dry solid. The term "tocopherol and/or an equivalent thereof", and 'alpha-TE', as used in this description, comprises tocopherols, tocotrienols, pharmaceutical and/or nutritional acceptable derivatives thereof and any combination thereof. The above numbers are based on tocopherol equivalents, recognized in the art.

Preferably the composition comprises between 0.01 and 1 mg selenium per serving, preferably between 0.02 and 0.2 mg per serving. Alternatively, preferably the composition comprises between 0.03 and 3 mg selenium per 100 g, preferably between 0.06 and 0.6 mg per 100 g dry solid. The amount of selenium administered per day is preferably 0.01 - 1 mg, more preferably 0.02 - 0.2 mg per day.

A preferred composition according to the invention comprises, per daily dose or per serving:
100 - 500 mg, preferably 200-400 mg EPA,
900 - 1500 mg, preferably 1000-1400 mg DHA,
50 - 600 mg, preferably 60-200 mg phospholipids,
200 - 600 mg, preferably 300-500 mg choline,
400 - 800 mg, preferably 500-700 mg UMP (uridine monophosphate),
20 - 60 mg, preferably 30-50 mg vitamin E (alpha-TE),
300 - 700 mg, preferably 400-600 mg vitamin C,
40 - 80 µg, preferably 50-70 µg selenium,
1 - 5 µg, preferably 2-4 µg vitamin B12,
0.5 - 3 mg, preferably 0.5-2 mg vitamin B6, and
200 - 600 µg, preferably 300-500 µg folic acid,
and wherein a serving preferably is 20 - 40 g dry solid, and/or wherein the composition is preferably administered once a day.

Vitamin A is advantageously administered in an amount of 0.05 - 2 mg, preferably 0.1 - 1 mg, more preferably 0.1 - 0.5 mg per serving or per day. Alternatively, vitamin A is preferably administered in an amount of 0.15 - 6 mg, preferably 0.3 - 3 mg, more preferably 0.3 - 1.5 mg per 100 g dry solid. Doses are given based on retinol activity equivalents (RAE), and the skilled person is capable to determine the dose of a retinol equivalent. Retinol activity equivalent is typically defined as 1 microgram RAE being equivalent to 1 microgram retinol or 12 microgram beta-carotene.

Vitamin D, also known as calciferol, comprises a group of fat-soluble secosteroids responsible for enhancing intestinal absorption of calcium, iron, magnesium, phosphate and zinc. In humans, the most important compounds in this group are vitamin D3 (also known as cholecalciferol) and vitamin D2 (ergocalciferol). Cholecalciferol and ergocalciferol can be ingested from the diet and from supplements. Any functional form of vitamin D known in the art is suitable to be used, including vitamin D1, vitamin D2, vitamin D3, vitamin D4, vitamin D5, or any combination thereof. Preferably, the composition comprises vitamin D2 and/or vitamin D3, more preferably vitamin D2. Vitamin D is advantageously administered in an amount of 0.1 - 10 µg, preferably 0.2 - 5 µg, more preferably 0.5 - 2 µg per serving or per day. Alternatively, vitamin D is preferably administered in an amount of 0.3 - 30 µg, preferably 0.6 - 15 µg, more preferably 1.5 - 6 µg per 100 g dry solid.

Phospholipids are in a preferred embodiment present in the composition by the addition of lecithin, suitably 0.01-1 g of lecithin per serving or per day. Lecithin is in a more preferred embodiment derived from soy. The composition preferably comprises between 0.01 and 1 gram lecithin per serving or per day, more preferably between 0.05 and 0.5 gram lecithin per serving or per day. The composition preferably comprises between 0.03 and 3 gram lecithin, more preferably between 0.15 and 1.5 gram lecithin per 100 g dry solid. In one embodiment, the phospholipids comprise at least two phospholipids selected from the group consisting of phosphatidylcholine, phosphatidylethanolamine, phosphatidylinositol and phosphatidylserine, preferably at least PC and PE.

Preferably carbohydrates are present in an amount of 10-50 g per serving or per day.

### Administration, dosing of composition and components

The composition is preferably provided in the form of a free-flowing powder comprised in a sealable can or box with accompanying reconstitution guidelines or instructions on the package. In a preferred embodiment, the composition of the invention is available in a box from which it is easy to dose the amount required for a serving, for example by scooping. With the box reconstitution instructions are provided for determining the concentration of the composition in water or another drink in a serving. The composition preferably comprises the active ingredients in predetermined serving amount which provides the actives in a daily recommended amount or as a part (such as ½ or 1/3) of a daily recommended amount.

In another embodiment, a serving of the composition is provided in the form of physically identifiable separate dosage units. One or more unit doses containing at least the active ingredients (i) - (iii) may be comprised in a cartridge, pack or dispenser device. Such a cartridge, pack or dispenser device can be accompanied by instructions for administration of the composition. A dosage regimen may involve the administration of one or more physically identifiable separate dosage units per serving, typically 1 - 5 unit doses per serving.

In one embodiment, the composition is provided in powder form comprised in a sachet, wherein the active ingredients (at least (i) - (iii)) are preferably present in a predetermined amount which, upon reconstitution following the guidelines or instructions accompanying the sachet, result in the adult human being provided with therapeutically effective amounts. It is preferred to provide the person with 1-3 unit doses (i.e. 1-3 sachets) per day. Suitable amounts are sachets that contain an amount of between 10 and 50 grams of the powder that can be added to a liquid one to three times a day, for example during or as part of a meal. In a preferred embodiment, a sachet comprises the daily dosage unit (providing the daily dosage of the actives). The invention also relates to a kit of parts comprising a plurality (e.g. 10 - 100) of servings in the form of physically identifiable, separate dosage units, preferably in the form of sachets. The sachets are preferably enclosed in a single packaging (e.g. carton, box, net or foil) and/or (mutually) physically interconnected by means of connecting portion(s) which are preferably breakable. The kit may be accompanied by instructions for use (reconstitution instructions).

### Process to prepare composition

The process of preparing the solid composition of the invention can take place by either dry blending the components (i) to (v), and optionally the further components, in dry form, or by bringing the components (i) to (v), and optionally the further components, together in an aqueous solution and subsequently drying the obtained solution by a drying step. The drying step is preferably an evaporation or spray or freeze drying step. In a preferred embodiment the components (i) to (v) and optionally one or more of the further components are combined in a dry blending step.

The process may contain an additional step in which the particle size of all components that are blended is uniformized, for example, by grinding larger particles into smaller ones or alternatively by compressing smaller particles into larger ones.

### Use of composition

The invention also covers the composition of the present invention for use in in adult humans to delay or prevent the onset of dementia, such as Alzheimer's Disease. Prophylactic treatment includes reducing the risk of occurrence of the disease or decline.

It is suited for use in adult humans to delay or prevent the onset of dementia, such as Alzheimer's disease, in particular in humans that are having symptoms that match being prodromal and/or have AD biomarker levels such as those for instance described in Soininen et al. "36-month LipiDiDiet multinutrient clinical trial in prodromal Alzheimer's disease" Alzheimer's Dement, 2020; 1 - 12 (XP055896952), or that have a mini mental state exam score (MMSE) of 20 or higher (i.e. of 20-30), preferably a MMSE score of at least 24, more preferably an MMSE score of at least 26. Mini mental state exam (MMSE) refers to a 30 point questionnaire to measure cognitive impairment such as in the diagnosis and longitudinal assessment of Alzheimer's disease. In the context of the invention, any score greater than or equal to 26 points (out of 30) indicates a normal cognition, i.e. e healthy adult person. Below this, scores can indicate severe (≤9 points), moderate (10-18 points) or mild (19-23 points) cognitive impairment. The raw score may be corrected for educational attainment and age.

To support that the composition of the present invention can be suitably used in the above applications and/or to create an effect in the above-identified subjects, reference is made to WO200184961, WO2003041701, WO2009002145, WO2009002163, WO2009002164, WO2009002166, WO2012091571, WO2012125020, WO2012091548, WO2013066165, WO2013066167, WO2013129931, WO2017155395, WO2017155396, WO2019013615 and to Soininen et al. "36-month LipiDiDiet multinutrient clinical trial in prodromal Alzheimer's disease" Alzheimer's Dement, 2020; 1 - 12.

### Examples

### Example 1

The below composition was made by dry blending the mentioned components

| **Component** | **Weight % addition** |
|---|---|
| Encapsulated DHA (Vana^{®}-Sana DHA 11 IF (FrieslandCampina) | 36.4 |
| Skim milk powder | 21.8 |
| Maltodextrin | 17.7 |
| Sugar (sucrose) | 12.0 |
| Choline bitartrate | 3.2 |
| Uridine 5 monophosphate | 3.1 |
| Flavour components | 2.8 |
| Potassium citrate | 0.72 |
| Vitamin premix | 0.66 |
| Soy lecithin | 0.62 |
| Magnesium acetate (4 hydrate) | 0.53 |
| Mineral premix | 0.47 |
| The pH of the composition was 5.3 | |

No phase separation or precipitation was observed and the sensory properties and reconstitution properties of the powder when dissolving it 30 grams in 125 ml of water were very good.

When in the above recipe one of the salts magnesium acetate or potassium citrate were left out (i.e there was no buffering system in the composition) or the protein source was a mix of calcium caseinate, sodium caseinate, and whey protein concentrate, one or more of phase separation, a precipitation and a fishy or otherwise unpleasant smell was observed when dissolving the powder in water.

When the recipe was repeated with choline chloride instead of choline bitartrate the storage stability was insufficient and the powder quality was evidently deteriorated after a 3 month storage. There was a high risk of clumping, high water activity in finished goods and handling conditions were intricate. Also off-flavours were noted which were attributed to the choline chloride.

### Example 2

A sealed box containing a free-flowing powder comprising:

| Serving size: Approximately 122 mL prepared | | | Average Quantity per Serving (30 g) | Amount per 100g solids, suited for 30 g serving |
|---|---|---|---|---|
| Servings per pack: 12 | | | | |
| Energy | | kcal | 133 | 443 |
| | | kJ | 556 | 1853 |
| Protein | | g | 3.3 | 11.0 |
| Carbohydrate | | g | 17.3 | 58 |
| - sugars | | g | 9.4 | 31 |
| | - lactose | g | 3.6 | 12.0 |
| Fat, total | | g | 5.5 | 18.3 |
| - phospholipids | | mg | 106 | 353 |
| - saturated | | g | 1.8 | 6 |
| - polyunsaturated | | g | 2.3 | 7.7 |
| | - Eicosapentaenoic acid (EPA) | mg | 300 | 1000 |
| | - Docosahexaenoic acid (DHA) | mg | 1200 | 4000 |
| - monounsaturated | | g | 1.2 | 4 |
| | | | | |
| Sodium | | mg | 200 | 667 |
| Potassium | | mg | 247 | 823 |
| Calcium | | mg | 82 | 273 |
| Phosphorus | | mg | 153 | 510 |
| Magnesium | | mg | 26 | 87 |
| Chloride | | mg | 63 | 210 |
| | | | | |
| Vitamin A | | µg-RE | 207 | 690 |
| Vitamin D | | µg | 1 | 3.3 |
| Vitamin E | | mg α-TE | 40 | 133 |
| Vitamin K | | µg | 7.9 | 26 |
| Vitamin C | | mg | 482 | 1607 |
| Thiamin (B₁) | | mg | 0.19 | 0.63 |
| Riboflavin (B₂) | | mg | 0.22 | 0.73 |
| Niacin (B₃) | | mg | 2.3 | 7.7 |
| Vitamin B₆ | | mg | 1 | 3.3 |
| Vitamin B₁₂ | | µg | 3 | 10.0 |
| Folic acid | | µg | 400 | 1333 |
| Pantothenic acid (B₅) | | mg | 0.67 | 2.2 |
| Biotin | | µg | 5 | 16.7 |
| | | | | |
| Choline | | mg | 400 | 1333 |
| Iron | | mg | 2 | 6.7 |
| Zinc | | mg | 1.5 | 5.0 |
| Manganese | | mg | 0.43 | 1.4 |
| Copper | | mg | 0.22 | 0.73 |
| Iodine | | µg | 20 | 66.7 |
| Molybdenum | | µg | 12.8 | 42.7 |
| Selenium | | µg | 60 | 200 |
| Chromium | | µg | 8.4 | 28 |
| | | | | |
| Uridine 5' monophosphate | | mg | 625 | 2083 |

The composition further comprises a buffering salt component with a buffer capacity in the range of pH 5 - 8.

### Example 3: powder tests

30 g of the composition as detailed in the table below (A) was reconstituted in 100 ml water. Variations in the recipe (B-E) were studied for impact on dissolution, and compared to a control recipe (without an uridine source):
B: effect of a mixture of 90:10 choline bitartrate and choline chloride;
C: effect of a mixture of 50:50 choline bitartrate and choline chloride;
D: effect of replacing skim milk powder with whey protein isolate; and
E: effect of replacing skim milk powder with caseinate;

For practical reasons, none of the recipes contained any uridine source. Examples 1 and 2 provide guidance as to suitable amounts of uridine sources.

Each time, directly after preparation, reconstitution in water was monitored in terms of dissolution (i.e. the time in seconds for the powder to dissolve) and lumping or caking behavior. The latter was studied using a 600 micron sieve, using a scale 0-5 (0: no lumps, 5: big lumps and/or several lumps present). The results are reported in the same table below.

**Table with powder dissolution tests; impact study on recipe variations**

| | | **90:10 mix of choline bitartrate: choline chloride** | **50:50 mix of choline bitartrate: choline chloride** | **Whey protein** | **Casein protein** |
|---|---|---|---|---|---|
| **Ingredient (amounts in grams)** | **A** | **B** | **C** | **D** | **E** |
| DHA (Vana^{®}-Sana DHA 11 IF (FrieslandCampina) 36.4 parts | 54.6 | 54.6 | 54.6 | 54.6 | 54.6 |
| Skim milk powder | 32.7 | 32.7 | 32.7 | 0 | 0 |
| Whey protein isolate | 0 | 0 | 0 | 32.7 | 0 |
| Caseinate | 0 | 0 | 0 | 0 | 32.7 |
| Choline bitartrate | 4.8 | 4.32 | 2.4 | 4.8 | 4.8 |
| Choline chloride | 0 | 0.48 | 2.4 | 0 | 0 |
| Potassium citrate | 1.08 | 1.08 | 1.08 | 1.08 | 1.08 |
| Magnesium acetate (4 hydrate) | 0.795 | 0.795 | 0.795 | 0.795 | 0.795 |
| Maltodextrin | 38.025 | 38.025 | 38.025 | 38.025 | 38.025 |
| Sugar (sucrose) | 18 | 18 | 18 | 18 | 18 |
| Total (g) | 150 | 150 | 150 | 150 | 150 |
| | | | | | |
| Dissolution time (seconds) | 18 | 24 | 23 | 19 | 28 |
| Lumping score (0-5) | 2 | 2 | 1 | 4 | 5 |

The dissolution time for composition (A) was 18 seconds for the powder made with skim milk powder, DHA, choline bitartrate, with a buffering salt comprising potassium citrate and magnesium acetate. Lumping was observed only to a small and acceptable extent (control). However, when using whey protein isolate (D) or caseinate (E) instead of skim milk, severe caking was observed. When replacing part of the choline bitartrate by choline chloride (90:10 mix with choline bitartrate, B), dissolution time increased significantly (from 18 to 24 seconds i.e. 33% increase). Such an extended reconstitution time hampers consumer's appreciation and ease of handling. Using a further increase in the relative amount of choline chloride (50:50 mix with choline bitartrate; C), caking issues were reduced, but it was observed during the experiment that the composition turned more hygroscopic, rendering processing more difficult. Given the fact that the reconstitution experiment was carried out directly after preparation, the extent to which this increased hygroscopicity would affect handling was still limited. However, it was anticipated that reconstitution after storage will be more challenging, especially over longer periods or in places where humidity is relatively high (high humidity climates, kitchens).

## Claims

1. A solid composition in powder form, the composition containing (i) a uridine source, wherein the amount of the sum of all uridine sources, calculated on the basis of the weight of the molar equivalent of uridine monophosphate (UMP), is 0.3-15 g per 100 g dry weight of the composition, (ii) a polyunsaturated fatty acid, preferably an omega-3 polyunsaturated fatty acid, (iii) choline bitartrate, (iv) a buffering salt component, and (v) a milk protein source, wherein at least 50% of the choline in the composition is provided by choline bitartrate, wherein the milk protein source is a milk powder, and the buffering salt component containing at least one food approved salt, with a buffering capacity between pH 5 - 8, the buffering salt component preferably containing a mixture of food-approved salts, or a food-approved salt and its conjugated acid or base, wherein the buffering salt component maintains the pH in the range of 5-8 when the solid composition is dissolved in 1 wt% in water.

2. The solid composition according to claim 1, wherein the polyunsaturated fatty acid is EPA, DHA and/or DPA, preferably EPA and/or DHA, most preferably at least DHA.

3. The solid composition according to claim 1 or 2, wherein the uridine source is one or more of uridine, or an uridine salt, wherein the salt is preferably a uridine phosphate salt, more preferably uridine, uridine diphosphate and/or uridine monophosphate.

4. The solid composition according to any one of the preceding claims, wherein the buffering salt component comprises one or more salts of the group of citrate salts, acetate salt and hydrogen phosphate salts.

5. The solid composition according to any one of the preceding claims, wherein the milk protein source is skim milk powder.

6. The solid composition according to any one of the preceding claims, wherein the composition comprises 1- 10 g protein per serving of 20 - 40 g dry mass, more preferably 2 - 6 g per serving of 20 - 40 g dry mass.

7. The solid composition according to any one of the preceding claims, wherein the composition comprises at least 0.3 g choline per 100 g dry weight of the composition.

8. The solid composition according to any one of the preceding claims, wherein the composition comprises more than 0.1 g choline per serving of 20 - 40 g dry mass, preferably 0.1 to 2 g choline per serving of 20 - 40 g dry mass, more preferably 0.2 to 2 g choline per serving of 20 - 40 g dry mass, most preferably 0.2 to 1 g choline per serving of 20 - 40 g dry mass.

9. The solid composition according to any one of the preceding claims, wherein at least 85 % of the choline in the composition is provided by choline bitartrate, and less than 5 % of the choline in the composition is provided by choline chloride.

10. The solid composition according to any one of the preceding claims, wherein the composition comprises an amount of the sum of all uridine sources (calculated taking the molar equivalent to the UMP amount) of 0.1-5 g, preferably 0.2-2.5 g, more preferably 0.25-1 g per serving of 20 - 40 g dry mass; and/or wherein DHA is administered in in an amount of at least 0.5 g per 100 g, more preferably 0.5 - 5 g per serving of 20 - 40 g dry mass, most preferably 0.75 - 2 g per serving of 20 - 40 g dry mass.

11. The solid composition according to any one of the preceding claims, wherein the composition is comprised in a can or box, or in a sachet.

12. The solid composition of any one of the preceding claims, wherein the composition contains the uridine source in an amount of 1 to 5 wt% on dry matter, and/or DHA and/or EPA in an amount of 2.5 to 25 wt% on dry matter, and/or the choline bitartrate in an amount of 1 to 5 wt% on dry matter.

13. The solid composition according to any one of the preceding claims, wherein the composition is suitable for adult humans to delay or prevent the onset of dementia, such as Alzheimer's disease.

14. A process for preparing the solid composition according to any one of the preceding claims, by either dry blending the components (i) to (v) in dry form, or by bringing the components (i) to (v) together in an aqueous solution and subsequently drying the obtained solution by a drying step, that is preferably an evaporation, spray or freeze drying step.

15. The solid composition according to any one of claims 1 to 13, for use in adult humans to delay or prevent the onset of dementia, such as Alzheimer's Disease.

## Patentansprüche

1. Feste Zusammensetzung in Pulverform, wobei die Zusammensetzung Folgendes enthält: (i) eine Uridinquelle, wobei die Höhe der Summe aller Uridinquellen, berechnet auf der Grundlage des Gewichts des molaren Äquivalents von Uridinmonophosphat (UMP), 0,3-15 g pro 100 g Trockengewicht der Zusammensetzung beträgt, (ii) eine mehrfach ungesättigte Fettsäure, vorzugsweise eine Omega-3-mehrfach ungesättigte Fettsäure, (iii) Cholinbitartrat, (iv) eine Puffersalzkomponente und (v) eine Milchproteinquelle, wobei mindestens 50 % des Cholins in der Zusammensetzung durch Cholinbitartrat bereitgestellt werden, wobei die Milchproteinquelle ein Milchpulver ist und die Puffersalzkomponente mindestens ein für Lebensmittel zugelassenes Salz mit einer Pufferkapazität zwischen pH 5 - 8 enthält, wobei die Puffersalzkomponente vorzugsweise eine Mischung aus für Lebensmittel zugelassenen Salzen oder ein für Lebensmittel zugelassenes Salz und seine konjugierte Säure oder Base enthält, wobei die Puffersalzkomponente den pH-Wert im Bereich von 5-8 hält, wenn die feste Zusammensetzung in 1 Gew.-% Wasser gelöst wird.

2. Feste Zusammensetzung nach Anspruch 1, wobei die mehrfach ungesättigte Fettsäure EPA, DHA und/oder DPA ist, vorzugsweise EPA und/oder DHA, am bevorzugtesten zumindest DHA.

3. Feste Zusammensetzung nach Anspruch 1 oder 2, wobei die Uridinquelle Uridin und/oder ein Uridinsalz ist, wobei das Salz vorzugsweise ein Uridinphosphatsalz ist, bevorzugter Uridin, Undindiphosphat und/oder Uridinmonophosphat.

4. Feste Zusammensetzung nach einem der vorhergehenden Ansprüche, wobei die Puffersalzkomponente ein oder mehrere Salze der Gruppe der Citratsalze, Acetatsalze und Hydrogenphosphatsalze umfasst.

5. Feste Zusammensetzung nach einem der vorhergehenden Ansprüche, wobei die Milchproteinquelle Magermilchpulver ist.

6. Feste Zusammensetzung nach einem der vorhergehenden Ansprüche, wobei die Zusammensetzung 1 - 10 g Protein pro Portion von 20 - 40 g Trockenmasse umfasst, bevorzugter 2 - 6 g pro Portion von 20 - 40 g Trockenmasse.

7. Feste Zusammensetzung nach einem der vorhergehenden Ansprüche, wobei die Zusammensetzung mindestens 0,3 g Cholin pro 100 g Trockengewicht der Zusammensetzung umfasst.

8. Feste Zusammensetzung nach einem der vorhergehenden Ansprüche, wobei die Zusammensetzung mehr als 0,1 g Cholin pro Portion von 20 - 40 g Trockenmasse umfasst, vorzugsweise 0,1 bis 2 g Cholin pro Portion von 20 - 40 g Trockenmasse, bevorzugter 0,2 bis 2 g Cholin pro Portion von 20 - 40 g Trockenmasse, am bevorzugtesten 0,2 bis 1 g Cholin pro Portion von 20 - 40 g Trockenmasse.

9. Feste Zusammensetzung nach einem der vorhergehenden Ansprüche, wobei mindestens 85 % des Cholins in der Zusammensetzung durch Cholinbitartrat bereitgestellt werden und weniger als 5 % des Cholins in der Zusammensetzung durch Cholinchlorid bereitgestellt werden.

10. Feste Zusammensetzung nach einem der vorhergehenden Ansprüche, wobei die Zusammensetzung eine Höhe der Summe aller Uridinquellen (berechnet unter Berücksichtigung des molaren Äquivalents der UMP-Menge) von 0,1-5 g umfasst, vorzugsweise 0,2-2,5 g, bevorzugter 0,25-1 g pro Portion von 20 - 40 g Trockenmasse; und/oder wobei DHA in einer Menge von mindestens 0,5 g pro 100 g verabreicht wird, bevorzugter 0,5 - 5 g pro Portion von 20 - 40 g Trockenmasse, am bevorzugtesten 0,75 - 2 g pro Portion von 20 - 40 g Trockenmasse.

11. Feste Zusammensetzung nach einem der vorhergehenden Ansprüche, wobei die Zusammensetzung in einer Dose oder Schachtel oder in einem Beutel enthalten ist.

12. Feste Zusammensetzung nach einem der vorhergehenden Ansprüche, wobei die Zusammensetzung die Uridinquelle in einer Menge von 1 bis 5 Gew.-% bezogen auf die Trockenmasse und/oder DHA und/oder EPA in einer Menge von 2,5 bis 25 Gew.-% bezogen auf die Trockenmasse und/oder Cholinbitartrat in einer Menge von 1 bis 5 Gew.-% bezogen auf die Trockenmasse enthält.

13. Feste Zusammensetzung nach einem der vorhergehenden Ansprüche, wobei die Zusammensetzung für erwachsene Menschen geeignet ist, um das Auftreten von Demenz, z. B. der Alzheimer-Krankheit, zu verzögern oder zu verhindern.

14. Verfahren zur Herstellung der festen Zusammensetzung nach einem der vorhergehenden Ansprüche, entweder durch Trockenmischen der Komponenten (i) bis (v) in trockener Form oder durch Zusammenbringen der Komponenten (i) bis (v) in einer wässrigen Lösung und anschließendes Trocknen der erhaltenen Lösung durch einen Trocknungsschritt, der vorzugsweise ein Verdampfungs-, Sprüh- oder Gefriertrocknungsschritt ist.

15. Feste Zusammensetzung nach einem der Ansprüche 1 bis 13 zur Verwendung bei erwachsenen Menschen zur Verzögerung oder Verhinderung des Auftretens von Demenz, z. B. der Alzheimer-Krankheit.

## Revendications

1. Composition solide sous forme de poudre, la composition contenant (i) une source d'uridine, où la quantité totale de toutes les sources d'uridine, calculée sur la base du poids de l'équivalent molaire de l'uridine monophosphate (UMP), est comprise entre 0,3 et 15 g par 100 g de poids sec de la composition, (ii) un acide gras polyinsaturé, de préférence un acide gras polyinsaturé oméga-3, (iii) du bitartrate de choline, (iv) un composant de sel tampon, et (v) une source de protéines de lait, où au moins 50 % de la choline présente dans la composition est fournie par le bitartrate de choline, où la source de protéines de lait est du lait en poudre, et le composant de sel tampon contient au moins un sel de qualité alimentaire, avec une capacité tampon comprise entre pH 5 et pH 8, le composant de sel tampon contenant de préférence un mélange de sels de qualité alimentaire, ou un sel de qualité alimentaire et son acide ou sa base conjugué(e), où le composant de sel tampon maintient le pH dans la plage de 5 à 8 lorsque la composition solide est dissoute à 1 % en poids dans de l'eau.

2. Composition solide selon la revendication 1, où l'acide gras polyinsaturé est l'EPA, le DHA et/ou le DPA, de préférence l'EPA et/ou le DHA, et de manière encore plus préférée au moins le DHA.

3. Composition solide selon la revendication 1 ou 2, où la source d'uridine est une ou plusieurs parmi l'uridine ou un sel d'uridine, où le sel est de préférence un sel phosphate d'uridine, plus préférablement l'uridine, l'uridine diphosphate et/ou l'uridine monophosphate.

4. Composition solide selon l'une quelconque des revendications précédentes, où le composant de sel tampon comprend un ou plusieurs sels choisis dans le groupe formé par les sels de citrate, les sels d'acétate et les sels d'hydrogénophosphate.

5. Composition solide selon l'une quelconque des revendications précédentes, où la source de protéines de lait est du lait écrémé en poudre.

6. Composition solide selon l'une quelconque des revendications précédentes, où la composition comprend de 1 à 10 g de protéines par portion de 20 à 40 g de matière sèche, de préférence de 2 à 6 g par portion de 20 à 40 g de matière sèche.

7. Composition solide selon l'une quelconque des revendications précédentes, où la composition comprend au moins 0,3 g de choline par 100 g de poids sec de la composition.

8. Composition solide selon l'une quelconque des revendications précédentes, où la composition comprend plus de 0,1 g de choline par portion de 20 à 40 g de matière sèche, de préférence de 0,1 à 2 g de choline par portion de 20 à 40 g de matière sèche, plus préférablement de 0,2 à 2 g de choline par portion de 20 à 40 g de matière sèche, et encore plus préférablement de 0,2 à 1 g de choline par portion de 20 à 40 g de matière sèche.

9. Composition solide selon l'une quelconque des revendications précédentes, où au moins 85 % de la choline dans la composition provient du bitartrate de choline, et moins de 5 % de la choline dans la composition provient du chlorure de choline.

10. Composition solide selon l'une quelconque des revendications précédentes, où la composition comprend une quantité totale de toutes les sources d'uridine (calculée en prenant l'équivalent molaire de la quantité d'UMP) de 0,1 à 5 g, de préférence de 0,2 à 2,5 g, plus préférablement de 0,25 à 1 g par portion de 20 à 40 g de matière sèche ; et/ou où le DHA est administré en une quantité d'au moins 0,5 g pour 100 g, de préférence de 0,5 à 5 g par portion de 20 à 40 g de matière sèche, et de manière encore plus préférée de 0,75 à 2 g par portion de 20 à 40 g de matière sèche.

11. Composition solide selon l'une quelconque des revendications précédentes, où la composition est contenue dans un récipient métallique ou une boîte, ou dans un sachet.

12. Composition solide selon l'une quelconque des revendications précédentes, où la composition contient la source d'uridine en une quantité de 1 à 5 % en poids par rapport à la matière sèche, et/ou du DHA et/ou de l'EPA en une quantité de 2,5 à 25 % en poids par rapport à la matière sèche, et/ou du bitartrate de choline en une quantité de 1 à 5 % en poids par rapport à la matière sèche.

13. Composition solide selon l'une quelconque des revendications précédentes, où la composition est adaptée aux humains adultes pour retarder ou prévenir l'apparition de la démence, telle que la maladie d'Alzheimer.

14. Procédé de préparation de la composition solide selon l'une quelconque des revendications précédentes, soit en mélangeant à sec les composants (i) à (v) sous forme sèche, soit en mélangeant les composants (i) à (v) dans une solution aqueuse, puis en séchant la solution obtenue par une étape de séchage, de préférence une étape d'évaporation, de séchage par atomisation ou de lyophilisation.

15. Composition solide selon l'une quelconque des revendications 1 à 13, destinée à être utilisée chez les humains adultes pour retarder ou prévenir l'apparition de la démence, telle que la maladie d'Alzheimer.
